# EUROPÄISCHE PATENTANMELDUNG

(11) **EP 1 148 120 A1**
(43) Veröffentlichungstag der Anmeldung: **24.10.2001**
(21) Anmeldenummer: 01108812.7
(22) Anmeldetag: 07.04.2001
(51) Int. Cl.: C12M 1/00, C12M 1/02, A01N 1/02, B01L 7/00

(54) **Vorrichtung und Verfahren zur Behandlung und Untersuchung von Lebewesen und Gewebe unter temperierten Bedingungen**

(30) Priorität: 22.04.2000 DE 10020072
(71) Anmelder: FORSCHUNGSZENTRUM JÜLICH GMBH, 52425 Jülich (DE)
(72) Erfinder: Pissarek, Margit, 52223 Stolberg (DE)

(57) **Zusammenfassung**

Die Erfindung betrifft eine Vorrichtung und ein Verfahren zur Behandlung und Untersuchung von Lebewesen und Gewebe unter temperierten Bedingungen.

Bei physiologischen und biochemischen Untersuchungen sowie präparativen Arbeiten an Lebewesen und Gewebe spielt das Aufrechterhalten einer konstanten Temperatur während der Untersuchung und Behandlung eine entscheidende Rolle für die Reproduzierbarkeit und Genauigkeit der Ergebnisse. Nach dem Stand der Technik wird entweder nur das Untersuchungsobjekt selbst temperiert, wodurch Schwankungen der Umgebungstemperatur zu ungenauen Ergebnissen führen, oder es wird eine temperierte Abdeckung über das Untersuchungsobjekt gestülpt, die jedoch weitere manuelle Eingriffe am Untersuchungsobjekt während der Untersuchung und Behandlung erschweren. Die erfindungsgemäße temperierte Abdeckung 3 mit Öffnung sowie die temperierte Unterlage 2 ermöglicht das Aufrechterhalten einer konstanten Temperatur in der Umgebung des Untersuchungsobjektes und eine Temperierung des Untersuchungsobjektes selbst. Gleichzeitig ist es möglich, Manipulationen am Untersuchungsobjekt während der Untersuchung und Behandlung ohne technischen Aufwand vorzunehmen.

## Beschreibung

Die Erfindung betrifft eine Vorrichtung sowie ein Verfahren zur Behandlung und Untersuchung von Lebewesen und Gewebe unter temperierten Bedingungen.

Um eine möglichst lange Vitalität isolierter Organe oder Gewebe zu gewährleisten, und daraus folgend eine lange Messung der physiologischen und biochemischen Eigenschaften zu gewährleisten, werden verschiedene Präparationstechniken angewandt. Hierzu gehört u. a. die Perfusionstechnik. Sie beinhaltet eine ausreichende Versorgung des untersuchten Objektes mit den erforderlichen Nährstoffen sowie eine ausreichende Versorgung der Zellen mit Sauerstoff und CO₂. Es werden möglichst physiologisch ähnliche Bedingungen wie im Körper nachgebildet. Die Zellen werden mit einer Nährlösung umspült und zur Sauerstoffversorgung z. B. mit einer H₂O₂ Lösung versetzt oder mit Sauerstoff angereicherter Nährlösung umspült. An Stelle der Adern und Lungenbläschen werden die Zellen durch das Umspülen mit Nährlösung oder die Infusion mit Nährlösung und sauerstoffreicher Nährlösung oder Begasung versorgt und die Lebensfähigkeit der Zellen aufrechterhalten. Gleichzeitig findet ein Abtransport von niedermolekularen Produkten statt, die von den Zellen gebildet wurden.

Neben einer Versorgung der isolierten Organe oder Gewebe mit Nährstoffen spielt auch die Temperatur eine wichtige Rolle. Physiologische und biochemische Untersuchungen sowie die Präparation von Organen und Gewebe benötigen konstante thermische Versuchsbedingungen für die Aufrechterhaltung reproduzierbarer Ergebnisse. Mit Hilfe der Perfusionstechnik kann zwar das Organ oder Gewebe über die Versorgung mit temperierter Perfusionslösung teilweise auf die gewünschte Temperatur gebracht werden, eine schwankende Umgebungstemperatur führt jedoch zu Meßwertschwankungen bzw. Meßfehlern. Eine Temperierung der Umgebungsluft trägt daher zur erheblichen Verbesserung der Meßgenauigkeit bei. Gegenwärtig erfolgt die Temperierung der Umgebungsluft durch temperierbare, doppelwandige Glasgefäße, die über das Untersuchungsobjekt gestülpt werden oder in die das Untersuchungsobjekt eingehängt wird. Nachteilig bei diesen Vorrichtungen ist jedoch, daß während der Messungen eine Manipulation am Untersuchungsobjekt nur durch komplettes Entfernen der temperierten Abdeckung möglich ist. Dies führt zu einer erschwerten technischen Handhabung, zu deutlichen Temperaturschwankungen während der Messung und zu Zeitverzögerungen, bis die gewünschte Temperatur sich wieder eingestellt hat. Besonders bei Arbeiten mit kurzlebigen Radionukliden kann dies zu erheblichen Beeinträchtigungen der Messungen führen.

Es ist daher die Aufgabe der Erfindung, eine Vorrichtung und ein Verfahren zu schaffen, wodurch Untersuchungen und Behandlungen von Organen und Gewebe unter konstanten, definierten Temperaturen mit gleichzeitiger Manipulation während der Behandlung und Untersuchung des Objektes möglich werden.

Ausgehend vom Oberbegriff des Anspruch 1 wird die Aufgabe erfindungsgemäß gelöst mit den im kennzeichnenden Teil des Anspruchs 1 angegebenen Merkmalen sowie mit den im kennzeichnenden Teil des Anspruchs 9 angegebenen Merkmalen.

Mit der erfindungsgemäßen Vorrichtung und dem Verfahren ist es nunmehr möglich, Organe und Gewebe bei einer konstanten, definierten Temperatur zu untersuchen und gleichzeitig eine gute Zugänglichkeit zum Untersuchungsobjekt während der Untersuchung zu ermöglichen.

Vorteilhafte Weiterbildungen der Erfindung sind in den Unteransprüchen angegeben.

In einer vorteilhaften Weiterbildung (Anspruch 2) bewirkt die konvexe Erhebung ein schnelles Abfließen unerwünschter oder verbrauchter Reaktionslösung und ermöglicht so ein sauberes Arbeiten. Besonders bei der Verwendung von Radionukliden ist die Hochlagerung des Untersuchungsobjektes auf der konvexen Erhebung von Vorteil, da die Entfernung der verbrauchten Radionuklide aus der Umgebung des Objektes hier besonders schnell erfolgt. Gleichzeitig ist eine optimale Temperierung und schonende Lagerung des Untersuchungsobjektes möglich.

In einer vorteilhaften Ausführung gemäß Anspruch 3 kann Meßzubehör stabil in der Vorrichtung fixiert werden. In die Halterung mit den Aussparungen können z.B. Kabel, Schläuche oder Perfusionskatheter eingehängt werden.

Durch die separate Thermostatierung (Anspruch 4) lassen sich unterschiedliche Temperaturen für die temperierten Elemente einstellen. Dadurch kann z. B. die Temperatur der Abdeckung höher liegen als die der Unterlage. So können Hindernisse der Wärmeübertragung, die sich durch die Luft innerhalb der Abdeckung ergeben, ausgeglichen werden.

Die doppelwandige Ausführung (Anspruch 5) ermöglicht ein gleichmäßiges Durchströmen der Abdeckung und Unterlage mit Temperiermittel. Weiterhin wird durch die Verwendung von chemisch inertem Material eine Verfälschung oder Beeinflussung der Ergebnisse verhindert.

Durch die vorteilhafte Ausführung gemäß Anspruch 6 läßt sich eine genaue Temperatursteuerung der temperierten Elemente durchführen.

Die Ausrichtung der Strömungsrichtung des Temperiermittels in Richtung der Öffnung der Abdeckung (Anspruch 7) ermöglicht zum einen eine luftblasenfreie Durchströmung der Abdeckung dadurch, daß das Temperiermittel von unten nach oben geleitet wird und so die aufsteigenden Luftblasen mit sich führt. Zum anderen kann die angewärmte bzw. die gekühlte Luft innerhalb der Abdeckung die eintretende nicht temperierte Raumluft durch die Öffnung heraus verdrängen.

Durch die Möglichkeit der Temperierung der Vorrichtung in einem Temperaturbereich von 0°C bis 60°C (Anspruch 8) kann eine Behandlung und Untersuchung von Objekten in einem breiten Temperaturspektrum durchgeführt werden. Für präparative Behandlungen eignet sich ein Temperaturbereich von 5°C bis 15°C, während sich für physiologische Untersuchungen ein Temperaturbereich von 20°C bis 37°C besser eignet. Die Temperatur läßt sich ohne Umbau der Vorrichtung auf die jeweilig erforderlichen Bedingungen einstellen. Ein Organ kann beispielsweise bei einer Temperatur von 10°C präpariert werden, und anschließend direkt bei einer Temperatur von 25°C physiologisch untersucht werden.

Die vorteilhafte Weiterbildung gemäß Anspruch 10 erlaubt durch das Hochlagern des Objektes auf der konvexen Erhebung ein kontaminationsfreies Arbeiten. Unerwünschte Reaktionslösungen fließen schnell ab und können entsorgt oder gesammelt werden, z. B. durch Ableiten oder Absaugen in ein Auffangreservoir. Besonders bei der Verwendung von Radionukliden ist eine schnelle Entfernung der verbrauchten Radionuklide aus der Umgebung des Objektes von besonderem Vorteil. Weiterhin kann eine schonende Lagerung mit gleichzeitiger Temperierung der Organe bzw. Gewebe durchgeführt werden.

Durch die Halterung auf der temperierten Unterlage (Anspruch 11) ist ein Verfahren möglich, das eine stabile Fixierung von Meßzubehör, wie z.B. den Einsatz von Kabeln, Schläuchen oder Perfusionskathetern zuläßt.

Die separate Thermostatierung (Anspruch 12) ermöglicht ein Verfahren, das die unterschiedliche Temperatureinstellung der beiden temperierten Elemente erlaubt. Dies bietet den Vorteil, daß Temperaturwiderstände, die z.B. durch die Umgebungsluft innerhalb der Abdeckung entstehen, durch eine höhere Temperierung der Abdeckung gegenüber der Unterlage überwunden werden können.

Mit Hilfe einer vorteilhaften Weiterbildung gemäß Merkmal aus Anspruch 13 ist es möglich, die temperierten Elemente mit Temperiermittel zu durchströmen und gleichzeitig ein kontaminationsfreies Arbeiten zu ermöglichen.

Die Temperierung der Elemente über externe Thermostate gemäß Merkmal aus Anspruch 14 erlaubt es, mit einer genauen Temperatursteuerung zu arbeiten, die auch eine Änderung der Temperatur über die Thermostate ermöglicht.

Durch die vorteilhafte Weiterbildung des Verfahrens gemäß Merkmal aus Anspruch 15 ist es möglich, die Veränderung der Temperatur im inneren Bereich der Abdeckung und im Gewebe oder Organ selbst durch Vermischen mit der untemperierten Raumluft zu verhindern. Dies ist durch das Verdrängen der untemperierten Luft durch die temperierte Luft durch die Öffnung der Abdeckung möglich.

Die vorteilhafte Ausführung gemäß Merkmal aus Anspruch 16 ermöglicht es, ein Verfahren zur Untersuchung und Behandlung von Lebewesen und Gewebe in einem Bereich von 0°C bis 60°C durchzuführen.

Die Zeichnungen zeigen beispielhaft eine Ausgestaltung der erfindungsgemäßen Vorrichtung.

Es zeigt:
- Fig. 1:: Eine Seitenansicht der Vorrichtung im Querschnitt
- Fig. 2:: Eine Frontansicht der Vorrichtung
- Fig. 3:: Eine Frontansicht auf die Vorrichtung im Querschnitt
- Fig. 4:: Eine Aufsicht auf die Vorrichtung im Querschnitt

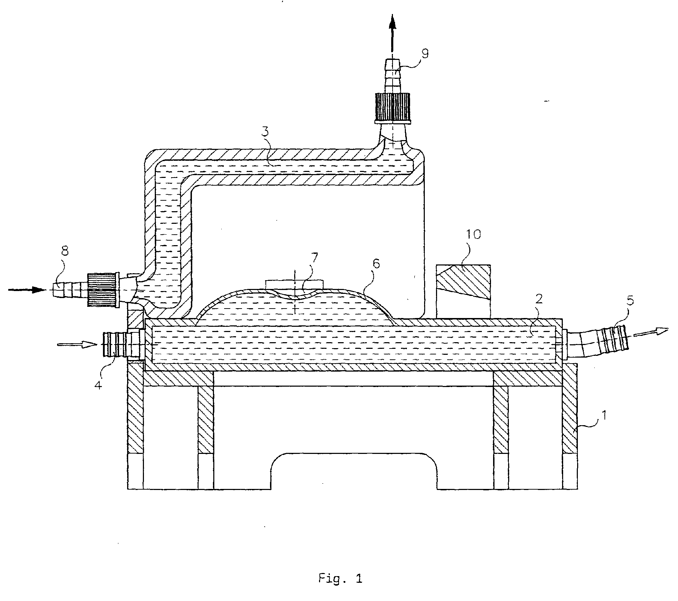

Die in Fig. 1 dargestellte Vorrichtung umfaßt ein Untergestell 1 zur Lagerung von zwei Temperierelementen 2 und 3. Dem Untergestell 1 liegt die temperierte Unterlage 2 auf, welche an den Kopfseiten zwei Anschlüsse 4 und 5 aufweist, die mit einem externen Thermostat verbunden werden. Die temperierte Unterlage 2 wird von links nach rechts in Richtung der Öffnung der oberen Abdeckung mit Temperiermittel, z.B. Wasser durchströmt. Die Unterlage weist eine konvexe Erhebung 6 auf, die zur Hochlagerung des Untersuchungsobjektes dient. In einer bevorzugten Ausführungsform weist die konvexe Erhebung 6 noch eine Einbuchtung 7 auf, die zu einer besonders stabilen Hochlagerung des Untersuchungsobjektes dient.

Auf die temperierte Unterlage 2 ist die temperierte Abdeckung 3 aufgebracht, welche mit Hilfe von Zapfen und entsprechenden Bohrungen auf der Unterlage 2 fixiert ist und daher leicht von der übrigen Vorrichtung abgehoben werden kann. Die temperierte Abdeckung 3 weist an der Rückwand und auf der Oberseite Anschlüsse 8 und 9 für die Verbindung zum externen Thermostat auf. Das Temperiermittel, z. B. Wasser, strömt von unten nach oben durch die temperierte Abdeckung 3. Die temperierte Unterlage 2 weist zusätzlich eine Halterung 10 auf.

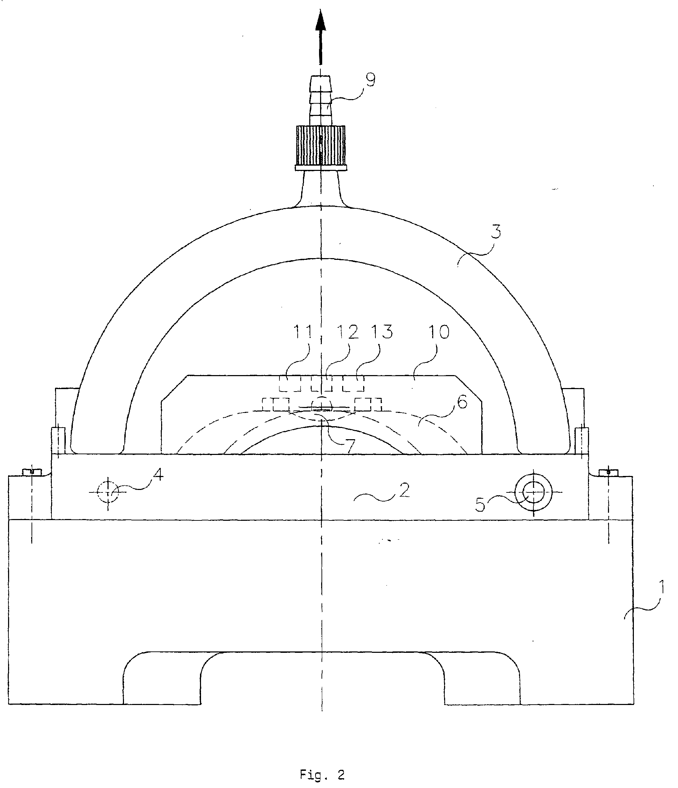

Figur 2 zeigt eine Frontansicht der Figur 1. In ihr sind denselben Vorrichtungsmerkmalen die gleichen Bezugszeichen zugeordnet. In der Frontansicht läßt sich erkennen, daß die temperierte Abdeckung 3 die Form einer Haube aufweist. Weiterhin erkennbar ist die Halterung 10 mit drei Aussparungen 11, 12, 13.

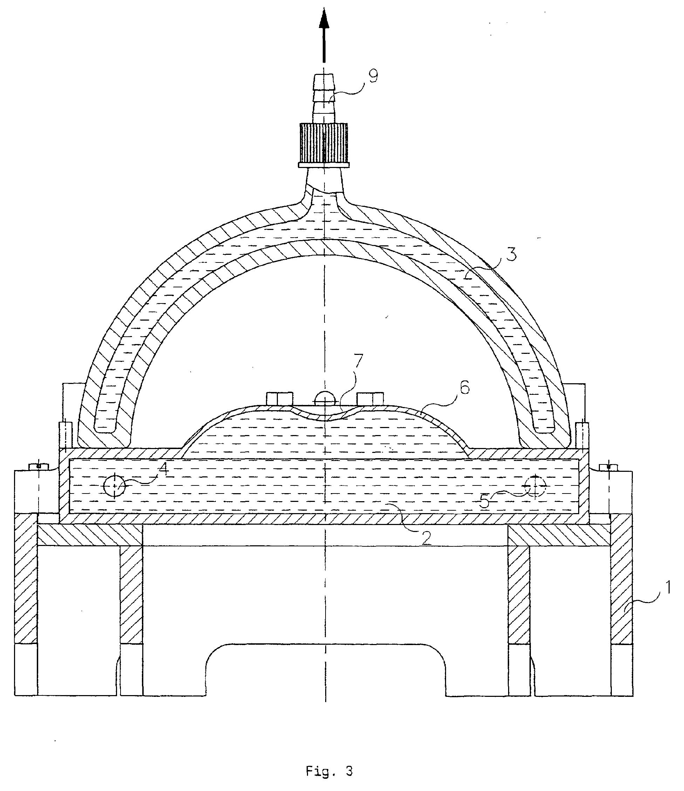

Figur 3 zeigt die Frontansicht auf Figur 1 im Querschnitt. In ihr sind denselben Vorrichtungsmerkmalen die gleichen Bezugszeichen zugeordnet. Im Querschnitt der Frontansicht ist die Füllung der temperierten Abdeckung 3 und Unterlage 2 mit Temperiermittel zu sehen.

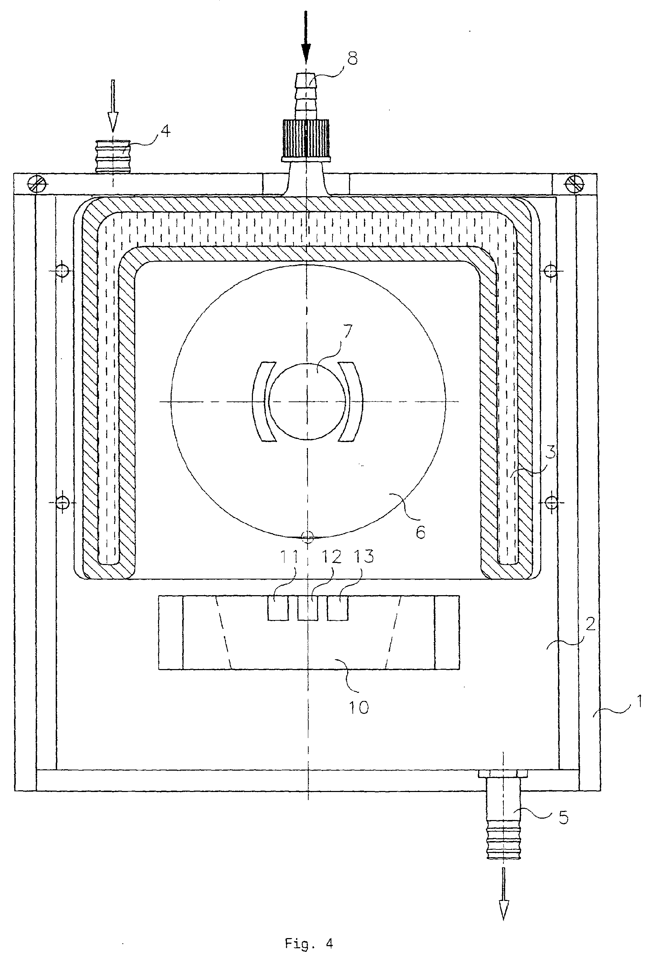

Figur 4 zeigt die Aufsicht auf Figur 1 im Querschnitt. In ihr sind denselben Vorrichtungsmerkmalen die gleichen Bezugszeichen zugeordnet. In der Aufsicht läßt sich erkennen, daß das Temperiermittel von der linken Ecke der Rückseite der Unterlage zur rechten Ecke der Frontseite der Unterlage geleitet wird.

Im folgenden soll die Erfindung beispielhaft erläutert werden.

Das Untersuchungsobjekt wird auf die temperierte Unterlage 2 gelegt. Anschließend wird die temperierte Abdeckung 3, die mit Hilfe von Zapfen und entsprechenden Bohrungen auf der Unterlage 2 fixiert werden kann, auf die Unterlage 2 gesetzt. Erforderliche Meßinstrumente, wie z.B. eine EEG-Meßapparatur, Mikrodialysesysteme oder adaptierte stereotaktische Einrichtungen können ebenfalls unter der Abdeckung 3 angeordnet werden. Die Öffnung der Abdeckung 3 ist so groß gewählt, daß ein bequemes Arbeiten am Untersuchungsobjekt möglich ist und gegebenenfalls Meßgeräte neu eingestellt bzw. ausgetauscht werden können. Dies führt zu keiner erheblichen Veränderung der Temperatur, da diese Veränderungen ohne Abnahme der temperierten Abdeckung 3 möglich sind. Auch aufwendige Veränderungen, wie z.B. Wechsel eines Meßgerätes können mit einer relativ geringen Temperaturänderung am Untersuchungsobjekts selbst vorgenommen werden. Die Abdeckung 3 kann ohne technischen Aufwand abgehoben werden. Durch die temperierte Unterlage 2 wird am Untersuchungsobjekt die Temperatur aufrechterhalten.

Das Material der temperierten Elemente der Vorrichtung besteht aus einem wärmeleitenden, chemisch inerten Material, wie z.B. Glas, PVC, PC und Polymethylmethacrylat. Um eine gute Überwachung des Untersuchungsobjekts zu gewährleisten, ist die Verwendung durchsichtiger Materialien zu bevorzugen. Als Temperiermittel wird bevorzugt Wasser eingesetzt. Je nach gewünschter Temperatur kann jedoch auch ein anderes Temperiermittel, welches sich speziell für Temperturen unter 0°C eignet, eingesetzt werden. Es können Temperaturen zwischen - 10°C bis 60°C eingestellt werden. Ein bevorzugter Temperaturbereich für physiologische Untersuchungen liegt bei 25°C bis 37°C. Für präparative Arbeiten wird ein Temperaturbereich zwischen 5°C bis 15°C bevorzugt. Beide Temperierelemente können getrennt voneinander thermostatiert werden, so daß beispielsweise die Temperatur der Abdeckung 3 höher liegen kann als die der Unterlage 2. Durch den direkten Kontakt des Untersuchungsobjektes mit der temperierten Unterlage 2 erfolgt eine schnelle Temperierung desselben. Die temperierte Unterlage 2 ist mit einer konvexen Erhebung 6 ausgestattet. Diese Erhebung 6 führt zu einem schnellen Abfließen unerwünschter oder verbrauchter Reaktionslösung und ermöglicht so ein sauberes Arbeiten. Die konvexe Erhebung ermöglicht weiterhin eine besonders schonende Lagerung der Untersuchungsobjekte. Eine Hochlagerung des Untersuchungsobjektes auf einem Gitter oder einem Netz würde ebenfalls zu einem schnellen Abfließen von Reaktionslösung führen, jedoch eine Schädigung des Gewebes infolge der rauhen, unebenen Oberfläche nach sich ziehen. In einer besonders bevorzugten Ausführungsform ist die Erhebung 6 mit Ablaufrinnen ausgestattet, die in ein Auffangreservoir münden und so zu einer besonders sauberen Entsorgung oder Sammlung von Reaktionslösung führt. In einer weiteren bevorzugten Ausführungsform weist die konvexe Erhebung eine Einbuchtung 7 auf, die zu einer stabilen Lagerung des zu untersuchenden Organs oder Gewebes führt. Die Einbuchtung 7 weist hierzu die gleiche Größe wie das Untersuchungsobjekt auf, wodurch ein Verrutschen des Objektes durch die Einbettung in die Einbuchtung 7 verhindert wird. Eine zusätzliche Fixierung kann beispielsweise durch das Einspannen des Untersuchungsobjektes zwischen Fixierbalken erfolgen.

Die Halterung 10 mit den Aussparungen 11, 12 und 13 dient der stabilen Befestigung von Meßzubehör, wie z.B. dem Einhängen von Schläuchen, Kabeln oder Perfusionskathetern.

Die erfindungsgemäße Vorrichtung eignet sich zur Untersuchung und Behandlung sowie der Präparation von Lebewesen, z.B. kleine Säuger, genauso wie zur Untersuchung und Behandlung sowie Präparation von isolierten Organen, wie z. B. Herz, Leber, Niere, Gehirn als auch Gewebe und einzelnen Zellen.

### Weiteres Ausführungsbeispiel:

Für die Untersuchung des Verhaltens von kurzlebig markierten Radiotracern an der Blut-Hirn-Schranke und für intrazerebrale Rezeptorbindungsstudien mit kurzlebig markierten Radiotracern bei isolierten Rattenhirnen werden zur Zeit die bilaterale und die unilaterale Perfusionstechnik eingesetzt.

Hierzu werden die Perfusionskatheter in der Aortenwurzel plaziert, während das Tier (nach Narkose und zervikaler Dislokation) in Rückenlage auf einem Eisbett gelagert ist.

Für die bilaterale Hirnperfusion wird der Perfusionskatheter nach Öffnung des Thorax beiderseits des Brustbeins in der Aortenwurzel fixiert. Die Arteria mammaria wird mit dem Elektrocouter verschlossen, und danach das Brustbein unterhalb des Verschlusses (knapp unter dem Manubrium sterni) entfernt. Die Aorta wird lateral der linken Arteria carotis communis verschlossen, die rechte Arteria subclavia lateral der rechten Arteria carotis communis. Anschließend werden die Gefäße im Halsbereich freigelegt, die Vena jugularis (rechts oder links) katheterisiert und die Äste der Arteria carotis externa und interna in dieser Region verschlossen. Danach wird die Kopf-Nackenregion vom Rumpf unter Erhaltung der Hirnperfusion abgetrennt und der Kopf so gedreht, daß er nun auf dem Unterkiefer gelagert ist.

Für die unilaterale Hirnperfusion wird der Katheter über die Aortenwurzel hinaus in die rechte Arteria carotis communis vorgeschoben, fixiert und dann für die Präparation der rechten Arteria carotis externa und interna vorgegangen, wie für die bilaterale Hirnperfusion beschrieben. Die linke Arteria carotis externa und interna werden für diese Variante verschlossen.

Das Perfusionsmedium wird während der Präparation in einer Langendorffapparatur mit Sauerstoff begast und auf 15°C gekühlt. Der Perfusionsfluß durch das Gehirn liegt zwischen 2 und 3 ml/min*g. Die Abdeckung 3 und die Unterlage 2 der erfindungsgemäßen Vorrichtung werden auf 15°C temperiert.

Der präparierte Kopf wird auf die konvexe Erhebung 6 der temperierten Unterlage 2 plaziert und die Perfusionskatheter in der Halterung 10 fixiert. Anschließend wird die temperierte Abdeckung 3 auf die Unterlage 2 gesetzt, so daß eine temperierte Kammer entsteht, die die Katheter bequem verlassen können und die für weitere Manipulationen durch den Experimentator gut zugänglich ist. Das verwendete durchsichtige Glas- bzw. Kunststoffmaterial erlaubt eine gute Kontrolle der Wasserfüllung der temperierten Elemente sowie des untersuchten Objektes und dort angebrachter Meßeinrichtungen.

Nach vollständiger Installation der Kammer wird die Temperatur innerhalb der Kammer von 15°C auf 37°C erhöht. Die Temperatur des Langendorffapparates und des Thermostates wird auf ca. 42°C eingestellt.

Die Installation der bilateralen Perfusion dauert zur Zeit 30 Minuten, die der unilateralen 20 Minuten. Die Zeit von der zervikalen Dislokation bis zur Plazierung der Perfusionskanüle beträgt maximal 2 Minuten.

Die erfindungsgemäße Vorrichtung erlaubt eine gut kontrollierbare Temperierung sowie durch die Hochlagerung des Hirns auf der Erhebung die Vermeidung unerwünschter Kontamination des Hirns durch den Tracer und die Gewinnung des Perfusats sowohl aus dem Jugularis-Katheter als auch nach Auffangen im Randbereich des Untergestells 1 durch Absaugen, Abpipettieren oder ähnliches. Mit der erfindungsgemäßen Vorrichtung wird eine schnelle Gewebsentnahme für biochemische Untersuchungen mittels Mikrobohrer und Öffnung des Schädels mittels Schere unter Erhaltung der Perfusion und der gewünschten Temperatur möglich. Auch der Einsatz von Frierstopptechniken ist mit dieser Vorrichtung gut möglich.

## Patentansprüche

1. Vorrichtung zur Behandlung und Untersuchung von Lebewesen und Gewebe unter konstanten thermischen Bedingungen,
**dadurch gekennzeichnet,**
**daß** sie eine temperierte Unterlage (2) sowie eine temperierte Abdeckung (3) mit Öffnung umfaßt.

2. Vorrichtung nach Anspruch 1,
**dadurch gekennzeichnet,**
**daß** die temperierte Unterlage (2) eine konvexe Erhebung (6) umfaßt.

3. Vorrichtung nach einem der Ansprüche 1 bis 2,
**dadurch gekennzeichnet,**
**daß** die Unterlage (2) eine Halterung (10) umfaßt.

4. Vorrichtung nach einem der Ansprüche 1 bis 3,
**dadurch gekennzeichnet,**
**daß** die temperierten Elemente jeweils separat thermostatiert sind.

5. Vorrichtung nach Anspruch 4,
**dadurch gekennzeichnet,**
**daß** die Elemente doppelwandiges, chemisch inertes Material umfassen.

6. Vorrichtung nach einem der Ansprüche 4 bis 5,
**dadurch gekennzeichnet,**
**daß** die Elemente über externe Thermostate kontinuierlich temperiert sind.

7. Vorrichtung nach einem der Ansprüche 1 bis 6,
**dadurch gekennzeichnet,**
**daß** die Strömungsrichtung des Temperiermittels in Richtung der Öffnung der Abdeckung (3) ausgerichtet ist.

8. Vorrichtung nach einem der Ansprüche 1 bis 7,
**dadurch gekennzeichnet,**
**daß** ein Temperaturbereich von 0°C bis 60°C innerhalb der Abdeckung (3) umfaßt wird.

9. Verfahren zur Behandlung und Untersuchung von Lebewesen und Gewebe unter konstanten thermischen Bedingungen
**dadurch gekennzeichnet,**
**daß** eine temperierte Unterlage (2) sowie eine temperierte Abdeckung (3) mit Öffnung verwendet wird.

10. Verfahren nach Anspruch 9,
**dadurch gekennzeichnet,**
**daß** eine temperierte Unterlage (2) mit einer konvexen Erhebung (6) eingesetzt wird.

11. Verfahren nach einem der Ansprüche 9 bis 10,
**dadurch gekennzeichnet,**
**daß** eine temperierte Unterlage (2) mit einer Halterung (10) eingesetzt wird.

12. Verfahren nach einem der Ansprüche 9 bis 11,
**dadurch gekennzeichnet,**
**daß** die temperierten Elemente jeweils separat thermostatiert werden.

13. Verfahren nach Anspruch 12,
**dadurch gekennzeichnet,**
**daß** Elemente aus doppelwandigem, chemisch inertem Material verwendet werden.

14. Verfahren nach einem der Ansprüche 12 bis 13,
**dadurch gekennzeichnet,**
**daß** die Elemente über externe Thermostate kontinuierlich temperiert werden.

15. Verfahren nach einem der Ansprüche 10 bis 14,
**dadurch gekennzeichnet,**
**daß** die Strömungsrichtung des Temperiermittels in Richtung der Öffnung der Abdeckung (3) ausgerichtet wird.

16. Verfahren nach einem der Ansprüche 10 bis 15;
**dadurch gekennzeichnet,**
**daß** innerhalb der Abdeckung (3) eine Temperatur von 0°C bis 60°C eingestellt wird.
